Europäisches Patentamt

European Patent Office

Office européen des brevets

(19)

(11) Publication number: **0 302 035**
**A2**

## EUROPEAN PATENT APPLICATION

(12)

(21) Application number: **88850252.3**

(22) Date of filing: **20.07.88**

(51) Int. Cl.⁴: **A 61 N 5/00**
**G 02 C 11/04**

(30) Priority: **29.07.87 SE 8702994**

(43) Date of publication of application:
**01.02.89 Bulletin 89/05**

(84) Designated Contracting States: **BE DE FR GB NL**

(71) Applicant: **Apoteksbolaget AB**
**S-105 14 Stockholm (SE)**

(72) Inventor: **Avén, Sven**
**Bällstavägen 45**
**S-161 71 Bromma (SE)**

(74) Representative: **Barnieske, Hans Wolfgang et al**
**c/o H.W. Barnieske Patentbyrä AB P.O. Box 25**
**Turingegatan 26**
**S-151 21 Södertälje 1 (SE)**

(54) A device for light treatment.

(57) A light-treating device for treating and preventing disturbing conditions, said device comprising a light source (1) and light-directing means (9, 10) to direct the light generated by the light source (1) into the wearer's eyes. The light is conducted from the light source (1) through optical conductors (2, 3, 4) and is reflected by reflectors (9, 10) into the eyes. The reflectors (9, 10) and light conductors (3, 4) are arranged on a holder resembling a spectacle frame which the patient wears while being treated.

FIG.1

Bundesdruckerei Berlin

## Description

### A device for light treatment

The invention relates to a device for treating and preventing disturbing conditions caused by a deficient supply of light.

Light therapy is a method of treatment which has recently come to the fore in alleviating and preventing certain seasonal diseases and problems such as autumn and spring depression. This type of problem occurs in a small group of people who are extra sensitive to light and who also react more strongly than others to shift work and to time adjustment when flying in east-west direction. The seasonal depression suffered by these people is probably caused by a disturbance in the hormone balance in the blood between cortisol which is a stress hormone secreted in the adrenal cortex, and melatonin which is a sleep-incuring hormone secreted in the pineal body. It is apparently sunlight which regulates the secretion of these hormones. Strong light impedes the production of melatonin. This is not particularly pronounced in most people, but the melatonin retardation appears to switch off immediately the supply of light decreases in the small group of light-sensitive people mentioned above, resulting in the difficulties mentioned.

An extra amount of artificial light to compensate for seasonal variations in the light of the sun has been found to have a positive effect both in preventing spring and autumn depressions and in treating those that have already broken out.

With the light treatment currently in use, the patient must remain in a special room with strong light intensity for an hour or so, once or twice a day during the autumn and winter months of the year.

This type of light therapy has been found to have beneficial effects in that the quantity of drugs administered in the traditional treatment can be greatly reduced or entirely omitted, but still it entails a number of not inconsiderable drawbacks. One such drawback is the considerable inconvenience to the patient in being tied to specific times and a special place for treatment. Another is that the treatment cannot be adapted to an individual if several patients are receiving light treatment simultaneously. The only parameter which can be varied is the duration of the treatment time. It is impossible to illuminate specific areas at the base of the eye, while others are not illuminated. Neither is it possible to adjust the wavelength interval or light intensity to individual requirements.

The object of the present invention is to eliminate the above-mentioned drawbacks and achieve a device which permits individual treatment, not tied to any place, which can be carried out at home, for instance, at a time of the patient's own choice.

This is achieved by the device comprising a holder, a light source and light-directing means connected to the holder, so arranged that when the holder is applied on the wearer said means direct the light generated in the light source into the wearer's eyes. This light-treatment device allows the wearer himself to select the time and place for treatment.

Furthermore, suitable treatment parameters such as wavelength interval and light intensity, can be adjusted individually for each patient.

The holder is suitably in the form of a spectacle frame.

The invention is described in more detail in the following, with reference to an embodiment shown by way of example in the accompanying drawings, in which

Figure 1 shows a means according to the invention in perspective, and

Figure 2 shows a detail modification of the invention, seen from the front.

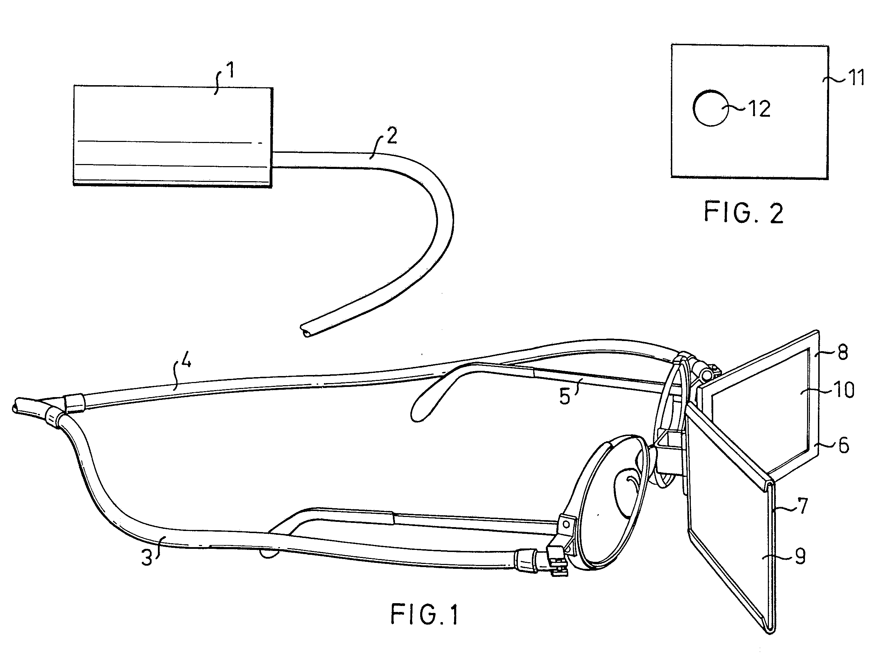

The apparatus for light treatment shown in Figure 1 comprises a light source 1 in which light of the desired type is generated. The light source 1 preferably includes means for adjusting the desired wavelength interval and light intensity. The light source 1 is connected to the main power supply, but it may also be supplied by battery and/or accumulator. An optical cable 2 conducts the light generated in the light source 1 via two branch leads 3 and 4 to a holder 5 in the form of a spectacle frame. The optical branch leads 3 and 4, one for each eye, are secured one to each side of the frame.

A holder 6 comprising two frames 7, 8, one for each eye, is secured to the nose section of the spectacle frame. A reflecting plate 9, 10 can be inserted in each frame 7, 8.

During the light therapy treatment the holder is applied on the nose like a pair of spectacles, with the shafts over the patient's ears. The frames 7, 8 and the ends of the optical cables 3 and 4 are thus directed so that the light conducted through the cables encounters the reflectors 9, 10 and is reflected into the patient's eyes.

The reflectors 9, 10 in Figure 1 are flat and whole, but other shapes are perfectly feasible. An example is shown in Figure 2 where the reflector 11 is provided with a through-hole 12. With such an arrangement, a certain area at the base of the eye will not be illuminated, which may be advisable in some cases.

Of course the treatment device need not be designed as shown here, although the spectacle form permits well-defined and constant positioning each time the device is used.

Furthermore, in the example the optical conductors are located on the outside of the shafts and are secured mechanically to the outside of the spectacle frame, but at least the end portions could be integrated (cast in) in the spectacle frame during manufacture.

Furthermore, the reflector plates 9, 10 may be domed in order to spread or concentrate the light in a specific manner into the eyes. They might also be coloured so that only certain wavelength intervals are reflected.

Preferably the reflector plates cover a significant portion of the patient's field of vision and are substantially uniformly illuminated. As shown in Fig. 1, the leads 3 and 4 may terminate at the forward end of the fore shaft ends, and then the reflectors may be arranged inclined at an angle of about 40°, for example.

The device could also be used to alleviate the inconvenience some people experience when flying in east-west direction. A study has indicated that many people suffer unpleasant depression if the time difference is greater than two hours. Such problems are probably due to the altered light conditions during the flight. Airlines could then provide light spectacles for people who react particularly strongly to the change during the journey.

**Claims**

1. A device for treating and preventing disturbing conditions caused by a deficient supply of light, **comprising** a holder (5), a light source (1) and light-directing means (9, 10) connected to the holder (5) so arranged that when the holder (5) is applied on the wearer said means direct the light generated in the light source (1) into the wearer's eyes.

2. A device as claimed in claim 1, **wherein** the directing means comprise reflectors (9, 10).

3. A device as claimed in claim 2, **wherein** the reflectors (9, 10) are removably and exchangeably arranged, each in its own frame (7, 8).

4. A device as claimed in claim 2 or 3, **wherein** each reflector consists of a flat plate (9, 10).

5. A device as claimed in claims 2 - 4, **wherein** each reflector consists of a curved surface.

6. A device as claimed in claims 2 - 5, **wherein** each reflector is provided with a through-hole (12).

7. A device as claimed in claims 1 - 6, **wherein** the holder (5) is in the form of a spectacle frame.

8. A device as claimed in claims 1 - 7, **wherein** the light generated by the light source (1) is conducted to the holder (5) by means of optical fiber 2, 3, 4).

0302035

FIG. 2

FIG.1